# EUROPEAN PATENT SPECIFICATION

(11) **EP 0 365 664 B1**
(45) Date of publication and mention of the grant of the patent: **05.04.1995**
(21) Application number: 89905904.2
(22) Date of filing: 28.04.1989
(51) Int. Cl.: A23K 1/00

(54) **USE OF SYNTHETIC L-CARNITINE IN FOOD FOR DOMESTIC DOGS OR CATS**
VERWENDUNG VON SYNTHETISCHEM L-CARNITIN IN HAUSHUND- ODER -KATZENFUTTER
UTILISATION DE L-CARNITINE DANS LA NOURRITURE DES CHIENS OU CHATS DOMESTIQUES

(30) Priority: 29.04.1988 US 187870
(43) Date of publication of application: 02.05.1990
(73) Proprietor: SHUG, Austin L., Madison, WI 53704 (US); KEENE, Bruce W., Raleigh, NC 27607 (US)
(72) Inventor: SHUG, Austin L., Madison, WI 53704 (US); KEENE, Bruce W., Raleigh, NC 27607 (US)
(74) Representative: SERJEANTS
(86) International application number: PCT/US89/01808
(87) International publication number: WO 89/10065

(56) References cited:
- WO-A-89/06958
- FR-A- 2 627 063
- JP-A-55 121 441
- US-A- 4 254 147
- US-A- 4 434 160
- US-A- 4 656 191
- US-A- 4 689 226
- US-A- 4 702 914

## Description

### General Field of the Invention:

The invention relates to the field of pet food compositions and more specifically to pet food enriched with L-Carnitine.

### Background of the Invention:

L-Carnitine is a quaternary amine that promotes beta-oxidation of long-chain fatty acids by facilitating their transfer across the mitochondrial membrane. L-Carnitine has also been shown to promote oxidation of branched-chain amino acids and the utilization of acetyl-coenzyme A.

In mammalian species, L-Carnitine concentration in cardiac and skeletal muscle is much higher than in serum. In these tissues fatty acids are utilized as a major source of energy. Because of L-Carnitine's central role in transporting fatty acids to the site of oxidation, adequate levels of L-Carnitine are required for normal fatty acid and energy metabolism in mammalian hearts. This is evidenced by the restoration to normal of fatty acid oxidation in muscle homogenates of certain L-Carnitine deficient patients. A relationship between deficient levels of myocardial L-Carnitine and cardiomyopathy has been observed in both hamsters and dogs. Restoration toward normal of such deficient L-Carnitine levels has been shown to result in improved myocardial function in both species.

In mammals, L-Carnitine is derived from the diet and from biosynthesis in the liver, and in some species, kidney and other tissues. Neither cardiac nor skeletal muscle is capable of synthesizing L-Carnitine, however. Thus, the L-Carnitine found in these tissues was either absorbed from the diet or biosynthesized endogenously by other tissues.

The invention is based on a study by the inventors of L-Carnitine levels in domestic dogs and cats, and a realization that the L-Carnitine levels regularly observed are substantially below optimum levels. The below optimum levels of L-Carnitine in domestic dogs and cats observed by the inventors are referred to herein as a diet-induced carnitine deficiency condition. This condition can lead to a multitude of conditions, including myopathic heart disease, ischemic heart disease, hyperlipidemia, ketosis, muscle weakness and premature aging.

The invention provides a use of commercially prepared synthetic L-Carnitine for the preparation of a nutritionally supplemented dog food or cat food for the alleviation of diet-induced carnitine deficiency in domestic dogs or cats.

The use according to the invention of commercially produced synthetic L-Carnitine for either a dietary supplement or a nutritionally supplemented dog or cat food is a veterinary use which prevents the above newly discovered condition in domestic dogs and cats.

The above research by the inventors has shown that pet dogs and cats are at great risk for developing L-Carnitine deficiencies. As Table 1 indicates, dog and cat foods are extremely low in free L-Carnitine levels as compared with that found in raw ground beef. Most pet dogs and cats are maintained strictly on commercial pet food diets and are thus kept chronically deficient in L-Carnitine. This results in the diet-induced carnitine deficiency.

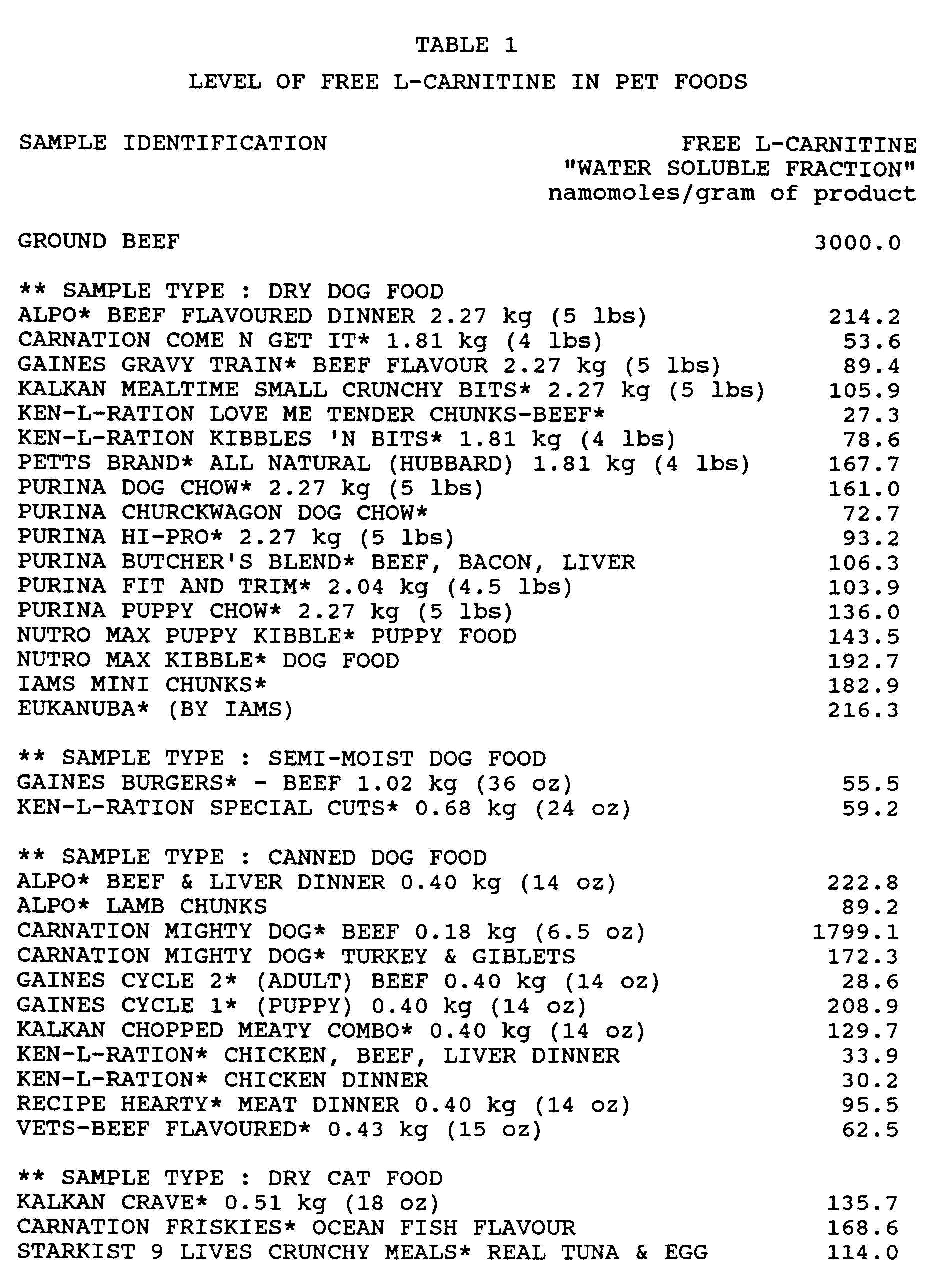

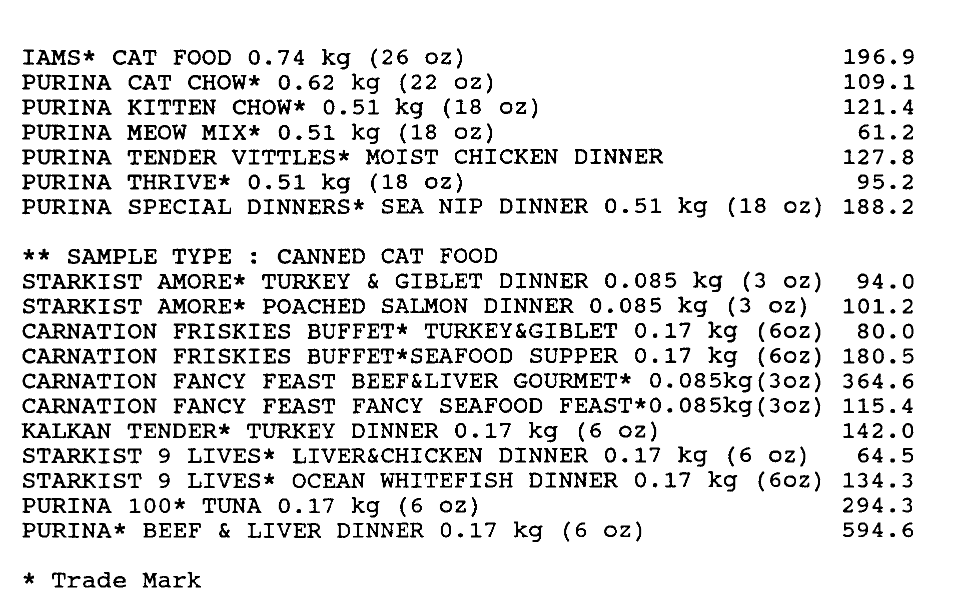

### Example

In this Example raw frozen lean ground beef was chosen as a convenient source of L-Carnitine in suitably high concentration. That choice is relevant for controlled experimental purposes only : it is well reported that raw or cooked meat alone is not a suitable diet for domestic dogs and cats. Meat is inadequate in calcium, phosphorus, sodium, iron and vitamins A, D and E, and provides an unhealthy amount of excess protein. Meat should not be fed raw because of the danger of transmitting parasites. Nevertheless the use of meat in this Example does illustrate the advantages that can be obtained according to the invention by substantially increasing the L-Carnitine content of an otherwise balanced diet of a domestic pet.

Six apparently healthy Greyhound dogs were determined to be normal by physical examination, fecal flotation, complete blood count, serum biochemical profile, ECG, and echocaridography. They were fed a standard commercial dog food diet free choice for a one-month control period. Control plasma samples (as well as subsequent test samples) were obtained following an eight-hour fast on two consecutive days for analysis of total, free, and esterified L-Carnitine concentration. The average of the plasma L-Carnitine concentration on two consecutive days was taken for each dog and each measuring period.

Following the control period, all of the dogs were continued for two weeks on the standard commerical dog food diet supplemented with L-Carnitine. The L-Carnitine supplement was in the form of 0.5kg per dog per day of raw frozen lean ground beef. This was equivalent to a daily supplement of 350 mg. of L-Carnitine per dog. Plasma samples were drawn on days 7 and 8 (averaged for the one-week measurement) and days 13 and 14 (averaged for the two-week measurement) for L-Carnitine analysis. Differences between the means of each test period and control were determined by the Student's t test.

### Results

The results of the study are shown in Table 2.

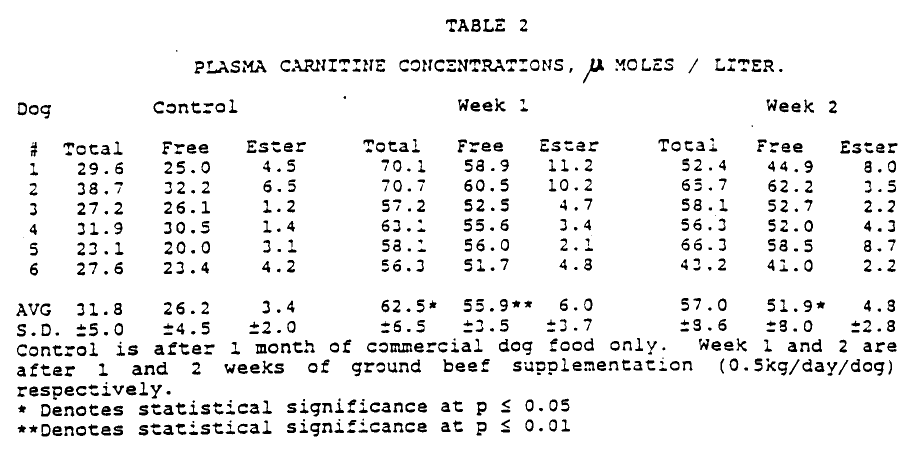

The data in Table 2 indicates that the plasma L-Carnitine concentration of a normal, otherwise healthy dog, previously maintained on a commercial pet food diet, is substantially deficient in carnitine as compared with the plasma carnitine levels found in other mammals. For example, in humans the mean value of plasma total carnitine is 59.3 ± 11.9 »M for males and 51.5 ± 11.6 »M for females. C.J. Rebouche and D.J. Paulson, Carnitine Metabolism and Function in Humans, 6 Ann. Rev. Nutr. pp. 41-66, at page 45. In rats, plasma carnitine concentration averages 56.5 ± 2.2 »M. P.R. Borum, "Regulation of the Carnitine Concentration in Plasma" in Carnitine Biosynthesis, Metaboiism, and Functions, 1980, Academic Press, New York, at page 119.

Further, the data of Table 2 indicates that the plasma total L-Carnitine concentration is significantly increased if the animal's diet is supplemented with L-Carnitine and that such level stabilizes in a range that is considered normal when compared with the plasma carnitine levels of other mammals. See Rebouche, supra, and Borum, supra.

It is clearly evident from the foregoing data that supplementation with a prophylactic amount of L-Carnitine of the standard commercial dog food will dramatically increase the plasma concentration of L-Carnitine in dogs. A prophylactic amount is the amount of L-Carnitine required to prevent the animal from developing a diet-induced carnitine deficiency. For a carnivore, such as a dog or cat, the preferred L-Carnitine content is advantageously roughly equivalent to the amount of L-Carnitine the animal would ingest if its diet consisted of red meat, i.e., at least approximately 700 mg of L-Carnitine per kilogram of food consumed.

It is understood that although the foregoing Example details the use of raw frozen lean ground beef as an L-Carnitine source, the invention envisages the provision of a dietary supplement or a pet food with an enhanced L-Carnitine content, either of which would provide essentially equivalent results. Also within the scope of the claims would be the use of commercially prepared L-Carnitine such as that obtained from Austin Chemical Company, Inc., 9655 West Bryn Mawr Avenue, Rosemont, Illinois.

These L-Carnitine supplements may be administered separately in the form of dietary supplements or they may be added at the time of manufacture of the commercial dog food as an additional ingredient in the commercial dog food. If used as a separate dietary supplement, the L-Carnitine may be combined with other valuable nutritional or prophylactic substances. Examples of this would be a combination of L-Carnitine with a vitamin and mineral preparation. Another example would be the inclusion of a prophylactic amount of L-Carnitine with an anti-heartworm medication such as diethyl-carbamazine.

The L-Carnitine supplement may also be administered as a liquid preparation. L-Carnitine is extremely soluble in water. Such a liquid preparation may be prepared by dissolving the appropriate amount of L-Carnitine in a waterbased solution. Flavoring agents or other nutritional or prophylactic substances may likewise be combined in the solution. The liquid preparation may be administered to the pet separately as a dietary supplement. It may be added to the pet's drinking water or to the animal's food. Further, the concentration of L-Carnitine in the liquid preparation may be such that it may be easily measured out and the prophylactic amount administered to the animal daily.

## Claims

1. Use of commercially prepared synthetic L-Carnitine for the preparation of a nutritionally supplemented dog food or cat food for the alleviation of diet-induced carnitine deficiency in domestic dogs or cats.

2. Use of commercially produced synthetic L-Carnitine according to claim 1, wherein the resulting dog food or cat food contains at least about 700 mg L-Carnitine per kilogram of the pet food.

## Patentansprüche

1. Verwendung von handelsüblichem synthetischem L-Carnitin zur Herstellung eines mit einem Futtermittelzusatz versetzten Hundefutters oder Katzenfutters zur Linderung eines diät-induzierten Carnitin-Mangels bei Haushunden oder -katzen.

2. Verwendung von handelsüblichem synthetischem L-Carnitin nach Anspruch 1, wobei das sich ergebende Hunde- oder Katzenfutter wenigstens etwa 700 mg L-Carnitin pro kg Haustierfutter enthält.

## Revendications

1. Utilisation de L-carnitine synthétique préparée commercialement pour la préparation d'un aliment pour chien ou chat supplémenté en élément nutritionnel en vue de pallier le déficit en carnitine d'origine alimentaire chez les chiens ou les chats domestiques.

2. Utilisation de L-carnitine synthétique produite commercialement selon la revendication 1, dans laquelle l'aliment pour chien ou chat ainsi préparé contient au moins 700 mg environ de L-carnitine par kilogramme d'aliment pour animal de compagnie.
